# FASCICULE DE BREVET EUROPEEN

(11) **EP 4 153 603 B1**
(45) Date de publication et mention de la délivrance du brevet: **01.11.2023**
(21) Numéro de dépôt: 21726422.5
(22) Date de dépôt: 20.05.2021
(51) Int. Cl.: C07F 7/18, A61K 8/58, A61Q 19/00, A61Q 19/08

(54) **COMPOSITIONS COMPRENANT DES COMPOSÉS ORGANO-SILANOLS, ET APPLICATIONS**
ZUSAMMENSETZUNGEN MIT ORGANOSILICIUMVERBINDUNGEN UND ANWENDUNGEN
COMPOSITIONS COMPRISING ORGANO-SILANOL COMPOUNDS, AND APPLICATIONS

(30) Priorité: 20.05.2020 FR 2005373
(43) Date de publication de la demande: 29.03.2023
(73) Titulaire: Exsymol, 98000 Monaco (MC)
(72) Inventeur: BONDON, Pierre, 98000 Monaco 4, avenue Albert II 98000 Monaco (MC)
(74) Mandataire: Murgitroyd & Company
(86) Numéro de dépôt international: PCT/EP2021/063565
(87) Numéro de publication internationale: WO 2021/234130

(56) Documents cités:
- US-A- 5 037 803
- US-A1- 2011 061 567

## Description

### Domaine technique

La présente invention concerne le domaine des compositions comprenant des composés organo-silanols stabilisés et biodisponibles, ainsi que leurs applications.

### Etat de la technique

Parmi les éléments chimiques identifiés dans la croûte terrestre, partie superficielle et solide de la Terre, le silicium se place au second rang après l'oxygène, avec, en masse, près de 28% de sa composition. Toutefois, ce métalloïde n'y figure pas à l'état libre et élémentaire de corps pur. En effet, et de par une très forte réactivité entre les deux éléments, le silicium se combine à l'oxygène ambiant pour générer une multitude d'oxydes, tels la très abondante silice (dioxyde de silicium, [SiO₂]) ou encore diverses structures tétraédriques [SiO₄⁴⁻] réunies sous la désignation de "silicates" et polymérisées et/ou associées à un ou plusieurs cations alcalins ou métalliques (Martin K.R., Met. Ions Life Sci., 2013, vol.13, pp.451-473). Ainsi, l'élément silicium, sous de telles formes pluri-atomiques, généralement solides et présentant un état cristallin ou amorphe, insolubles dans l'eau, s'avère être présent partout dans les sols de la Terre, en particulier dans nombre de roches aussi courantes que le grès, le granite, l'argile, le sable, etc. Plus de 90% en poids du paysage minéral de la croûte terrestre serait d'ailleurs composé de telles structures siliciées, regroupées en l'espèce sous le terme générique de "silice lithogénique" (R. Jugdaohsingh, J. Nutr. Health Aging, 2007, vol.11, pp.99-110). Avec une telle géologie, l'eau naturelle, de surface et de puits, affiche elle aussi du silicium dans sa composition, selon plusieurs formes hydrosolubles rassemblées sous une autre désignation collective, celle d'"acide silicique" pour réunir la forme monomère représentée par l'acide orthosilicique [H₄SiO₄] et des formes oligomères hydratées que sont principalement les acides méta- [H₂SiO₃], di- [H₂Si₂O₅] et tri-siliciques [H₂Si₃O₇] (Martin K.R., J. Nutr. Health Aging, 2007, vol.11, pp.94-9 ; Jurkić L.M. et al., Nutr. Meta., 2013, vol.10, pp.1-12).

Dans la matière constituant le vivant, à contrario du règne minéral, la prévalence générale du silicium n'est comparativement pas aussi marquée. Par exemple, seuls quelques organismes unicellulaires vivants présents dans les fonds marins sont capables d'accumuler du silicium, sous la forme d'une "silice biogénique", fraction de silice particulaire qu'ils fabriquent eux-mêmes et destinée notamment à assurer la robustesse de la cellule vivante, en particulier vis-à-vis des prédateurs, et à se protéger du rayonnement ultraviolet. Les diatomées, organismes unicellulaires planctoniques souvent associés aux microalgues, en constituent l'exemple le plus connu avec un endosquelette, ou frustule, en effet siliceux (Rabovsky J., Scand. J. Work Environ. Health, 1995, vol.21, pp. 108-110). Les plantes absorbent et concentrent elles aussi du silicium à partir des sols et des eaux qui en contiennent, à des fins de croissance et de résistance aux stress abiotiques auxquels elles sont exposées (variations climatiques, métaux lourds, etc.) et sous la forme, elles, d'une "silice colloïdale", à savoir une suspension de fines particules de silice et à des concentrations variables selon les espèces végétales, leur génotype, la phénophase de la plante, la saison, la nature du sol, etc. (Currie H.A. et al., Ann. Botan., 2007, vol. 100, pp.1383-1389 et références citées). Enfin quant à l'Homme, il naît avec un capital de silicium, qui s'élève au rang de troisième oligo-élément le plus abondant du corps humain après le fer et le zinc. Dans celui d'un adulte, la quantité de silicium varie pondéralement entre 1 et 2 grammes, avec une présence ubiquitaire dans les tissus et fluides biologiques (Götz W. et al., Pharmaceutics, 2019, vol.11, pp.1-27). Même s'il ne s'agit que d'un oligo-élément, et donc par définition d'une substance présente dans l'organisme en très faible quantité apportée exclusivement (le corps ne peut le fabriquer) par les nutriments, l'implication active du silicium dans la physiologie du corps humain n'est plus à démontrer avec des bénéfices apportés dans des mécanismes aussi variés que la minéralisation osseuse et la prévention de l'ostéoporose, la stimulation de la production de fibres protéiniques (collagène, élastine) et la prévention du vieillissement de la peau et des phanères, les risques réduits d'athérosclérose, de la maladie d'Alzheimer, la lutte contre les troubles de la pression artérielle, le stress oxydatif, l'inflammation, etc. (Martin K.R., Met. Ions Life Sci., 2013, vol.13, pp.451-473 et références citées). Son rôle est en particulier prépondérant dans la préservation de l'intégrité structurelle et métabolique des tissus conjonctifs et de soutien qui donnent forme et maintien à l'organisme, principalement tous les tissus qui nécessitent de la résistance et de la flexibilité tels ceux que l'on retrouve au niveau des muscles, de la peau, des os, des cartilages, des articulations, des tendons, des ligaments, des artères (Vasanthi N. et al., World Appl. Sci. J., 2012, vol. 17, pp. 1425-1440).

Il reste que, d'une part, le capital silicium chez l'Homme décroît naturellement, et de manière irréversible, avec l'âge à partir de la maturité sexuelle (Bissé E. et al., Annal. Biochem., 2005, vol.337, pp. 130-135). D'autre part, l'alimentation quotidienne et la consommation d'aliments et boissons réputés pour leur contenu en silicium (céréales complètes, haricots, banane, bière, etc.) peinent à combler un tel appauvrissement, en raison d'un silicium ingéré qui ne se présente pas en totalité sous une forme directement assimilable par le tractus gastro-intestinal, et l'incapacité du corps à stocker tout excédent potentiel. Enfin et de façon notoire avec la chimie du silicium, la seule forme naturelle d'un silicium réellement assimilable et biodisponible, représentée par l'acide orthosilicique [Si(OH)₄], affiche défavorablement une faible concentration dans l'eau (< 2 mM soit 56 mg Si/I) à cause d'une très grande tendance à s'auto-condenser dès que les teneurs en silicium s'élèvent, en conséquence à produire des formes polymérisées (en particulier via l'établissement de liaisons siloxanes (Si-O-Si) intermoléculaires, entre les fonctions silanols (Si-OH) portées par les molécules d'acide orthosilicique) peu, ou plus difficilement, absorbables par l'organisme et donc potentiellement inertes ou peu actives biologiquement (Jurkić L.M. et al., Nutr. Meta., 2013, vol.10, pp.1-12).

Afin de pouvoir remédier à de tels obstacles et difficultés d'assimilation du silicium, une avancée scientifique remarquable fut, à la fin des années 1950, objet des brevets FR1234213 et FR1069 et destinée à un usage thérapeutique, la création en synthèse organique d'un "silicium organique", dénommé ainsi au motif que sa structure affichait, par opposition au silicium minéral, un atome de silicium lié pour la première fois à un atome de carbone. Derrière cette expression plutôt triviale est visé un dérivé de silicium qui, par comparaison à la forme naturelle, l'acide orthosilicique, affiche un comportement dans l'eau largement supérieur (~ 21 mM soit 588 mg Si/I pour [CH₃-Si(OH)₃]), assimilable, stable, et structuralement de type "complexe organo-silicique" tel les titres des brevets susmentionnés. Chronologiquement, le premier "silicium organique" ainsi conçu le fut sous la forme de la molécule monomère monométhylsilanetriol [CH₃-Si(OH)₃] stabilisée par l'acide salicylique, qualifiée d'ailleurs historiquement de « *silicium organique de première génération* ». Puis se sont succédées d'autres versions avec, par exemple, la mise au point quelques temps après d'un « *silicium organique de seconde génération* » dans lequel l'acide citrique venait se substituer à l'acide salicylique en raison de patients allergiques aux dérivés salicylés (https://laurepouliquen.fr/quest-ce-que-le-silicium-organique/ & « L'aventure du silicium organique » Ambre Editions, 2010). En tout état de cause, tout au long des décennies qui ont suivi ces premiers développements, l'état de la technique s'est régulièrement enrichi de versions ou "générations" nouvelles de silicium organique stabilisé et assimilable, même si les finalités revendiquées se sont éloignées de l'usage thérapeutique originel. Illustrant cet aspect, l'on peut citer par exemple, à titre purement indicatif, les "silicium-cosmétiques" objets du brevet EP0289366, lesquels consistent, selon les inventeurs dudit brevet, en des complexes moléculaires de type hydroxysilanes destinés à constituer de nouveaux produits pour application cutanée ou capillaire, caractérisés par un atome de silicium porteur doublement, et d'un composé spécifique biologiquement actif, et d'une macromolécule (élastine hydrolysée, etc.) au caractère "dermatophile" et à l'action originale de fixer le silicium organique au niveau intra-cutané et donc d'éviter son passage dans les tissus sous-jacents de la peau. L'on peut citer également des travaux réalisés par la demanderesse avec la conception de complexes à base de composés organo-siliciés biologiquement actifs se présentant sous la forme originale d'une poudre afin de pouvoir intégrer des présentations cosmétiques et pharmaceutiques non aqueuses (comprimés, cachets), administrables par voie orale (brevet EP0867445). Plus proche de nous, l'on peut aussi citer, en réponse à une problématique cutanée spécifique, la conception en milieu aqueux de nouveaux complexes capables d'apporter à la peau un silicium biologiquement actif, constitués d'une entité organo-silanol, préférentiellement le méthylsilanetriol, associée et stabilisée par des liaisons faibles (liaisons hydrogènes) à des substances originales telles que des fragments calibrés d'un glycosaminoglycane (brevet EP2172186) ou encore une sélection de déoxy-sucres monomères (brevet FR3038898). L'on peut citer enfin, dans une littérature récente qui témoigne d'ailleurs de l'intérêt constant que suscite encore et toujours le silicium organique depuis sa création, deux autres exemples. Le premier d'entre eux, objet de la demande internationale WO2018/037115, concerne une composition présentée comme hautement concentrée en un complexe de silicium biodisponible, hautement assimilable, stable à un pH alcalin et combinant en phase aqueuse, un organosilanolate monomérique, préférentiellement le monométhylsilanolate, avec au moins un agent chélatant. Le second, lui objet d'un article, concerne un méthylsilanetriol associé et stabilisé par liaisons hydrogènes à deux substances structuralement d'intérêt pour leurs motifs phénoliques. Stabilité et biodisponibilité à des fins cosmétiques des complexes ainsi obtenus y sont particulièrement soulignées (Fastré M. et al., 2018, Chem. Sci. J., vol.9, pp.1-3). Des compositions cosmétiques sont décrites dans US 5 037 803 A. Par ailleurs, US 2011/061567 A1 décrit des fines particules à base d'oxyde métallique, ainsi que des compositions de résine.

Toutefois, il s'avère primordial de concevoir et mettre au point, encore et toujours, de nouvelles versions/ « générations » de silicium organique, plus stables, biodisponibles et biologiquement actives (en particulier en minimisant, le risque d'auto-condensation et de production de formes polymérisées et insolubles dans l'eau via l'établissement de liaisons siloxanes (Si-O-Si) intermoléculaires, tel qu'indiqué précédemment). La présente invention s'est inscrite dans cette recherche de nouvelles versions/générations de silicium organique, toujours plus stables, biodisponibles et biologiquement actives, notamment à destination de formulations cosmétiques et/ou dermocosmétiques et/ou nutricosmétiques buvables. En particulier, un des objectifs recherchés par la demanderesse a consisté à développer, dans un solvant polaire, une génération alternative et améliorée d'un silicium organique, possédant notamment une très grande stabilité dans le temps, hautement biodisponible et biologiquement actif (en particulier sur la peau y compris jusque dans ses couches les plus profondes).

### Exposé de l'invention

Dans un tel contexte de recherche visant à mettre au point une nouvelle génération de silicium organique répondant aux exigences susvisées, la demanderesse s'est attachée à améliorer les complexes ou compositions comprenant un organo-silanol associé à un ou plusieurs agents stabilisants/complexants. Toutefois, et en rupture avec l'approche traditionnellement suivie, au lieu d'axer ses recherches autour de la sélection du ou des agents stabilisants/complexants d'organo-silanols, la demanderesse a mené un travail autour de la solvolyse des organo-silanols *per se,* et plus précisément autour de ceux répondant à la formule générale (I) suivante : X-Si(OH)₃ (I) (dans laquelle le radical X est un groupement alkyle en C₁-C₄, linéaire ou ramifié, de préférence en C₁-C₂, éventuellement substitué par au moins un groupement hydroxyle), et en particulier autour du méthylsilanetriol, avec notamment pour objectif d'accroître la stabilité des systèmes comprenant ces organo-silanols stabilisés (au moyen du ou des agent(s) stabilisant(s)/complexant(s) susvisé(s)).

Ainsi, dans le cadre de ses recherches sur le mode de préparation en milieu aqueux desdits organo-silanols de formule générale (I), en particulier du méthylsilanetriol, la demanderesse a découvert, contre toute attente, qu'avec l'introduction d'un solvant glycolique de formule générale HO-CH(R₁)-CH(R₂)(R₃) (II) (dans laquelle R₁ est -H ou -CH₃ ; R₂ est -H, -OH, ou - CH₃ ; R₃ est -CH₃, -CH₂-CH₃, -CH₂OH, -CH(CH₃)OH, -CH₂-CH₂-CH₃, -CH₂-CH₂-CH₂-CH₃, - CH₂-CH₂-CH₂-CH₂-OH, ou -C(CH₃)(CH₃)-OH), l'on observait la transformation d'une fraction de l'organo-silanol de formule générale (I), tel que défini supra, en un autre organo-silanol de formule générale (III) suivante :

X-Si(OH)₂-O-CH(R₁)-CH(R₂)(R₃) (III)

dans laquelle :
- le radical X est un groupement alkyle en C₁-C₄, linéaire ou ramifié, de préférence en C₁-C₂, éventuellement substitué par au moins un groupement hydroxyle (de préférence par un groupement hydroxyle), de préférence X étant un groupement méthyle
- R₁ est -H ou -CH₃ ;
- R₂ est -H, -OH, ou -CH₃;
- R₃ est -CH₃, -CH₂-CH₃, -CH₂OH, -CH(CH₃)OH, -CH₂-CH₂-CH₃, -CH₂-CH₂-CH₂-CH₃, -CH₂-CH₂-CH₂-CH₂-OH, ou -C(CH₃)(CH₃)-OH.

La susdite transformation s'effectue via une réaction de monoalcoxylation entre le solvant glycolique retenu et l'un des groupements hydroxyle (portés par l'atome de silicium) de l'organo-silanol de formule générale (I), tel que défini supra.

Il résulte de cette transformation d'une fraction de l'organo-silanol de formule générale (I) en organo-silanol de formule générale (III), une coexistence des organo-silanols de formules générales (I) et (III). En outre, et de manière avantageuse, l'on note, au sein de l'organo-silanol de formule générale (III), l'occurrence d'une liaison chimique forte, en l'espèce une liaison covalente de type -Si-O-C-, élément chimiquement stabilisant et différentiant de l'organo-silanol de formule générale (I) stabilisé, comme dans l'art antérieur, par liaisons hydrogènes. A titre illustratif, si l'on considère spécifiquement le méthylsilanetriol en tant qu'organo-silanol de formule générale (I) et le 1 ,3-propanediol en tant que solvant glycolique de formule générale (II), l'on observe, en présence dudit 1,3-propanediol, la transformation d'une fraction d'un méthylsilanetriol en (3-hydroxypropoxy)-(méthyl)silanediol ; une des fonctions silanol du précurseur méthylsilanetriol se retrouvant stabilisée au sein du composé (3-hydroxypropoxy)-(méthyl)silanediol, non pas par une liaison hydrogène, comme dans l'art antérieur, mais bien par la formation d'une liaison covalente de type -Si-O-C-, issue d'une réaction de monoalcoxylation entre le 1,3-propanediol et l'un des groupements hydroxyle portés par l'atome de silicium du méthylsilanetriol.

De manière particulièrement avantageuse, en présence d'un ou plusieurs agents stabilisants/complexants (permettant la stabilisation des fonctions silanol via l'établissement de liaison(s) hydrogène(s) entre celles-ci et le/les agent(s) stabilisant(s)/complexant(s)), l'on observe, de manière tout à fait novatrice, dans des conditions opératoires appropriées (déterminables par l'homme du métier, sans difficulté excessive, sur la base de l'enseignement de la présente demande de brevet, complétée, le cas échéant, par ses connaissances générales) :
i) une dualité d'interactions chimiques stabilisantes, primordiales pour la stabilisation des fonctions silanols de l'organo-silanol de formule générale (I), avec précisément une présence concomitante de liaisons faibles (en l'espèce de liaisons hydrogène) avec le/les agent(s) stabilisant(s)/complexant(s) et de liaisons fortes (covalentes, de type -Si-O-C-, tel qu'indiqué supra), et
ii) la formation d'un complexe ternaire, stabilisé, entre l'organo-silanol de formule générale (I), le/les agent(s) stabilisant(s)/complexant(s) et l'organo-silanol de formule générale (III), pouvant être représenté schématiquement par :

   OS(I)---AS---OS (III)

   où :
   - « OS(I) » désigne l'organo-silanol de formule générale (I) tel que défini précédemment,
   - « AS » désigne, I'/les agent(s) stabilisant(s)/complexant(s) d'organo-silanols, tel(s) que défini(s) précédemment,
   - « OS(III) » désigne l'organo-silanol de formule générale (III) tel que défini précédemment, et
   - les traits pointillés représentant au moins une liaison hydrogène.

Les découvertes effectuées par la demanderesse ont permis la conception et la mise au point d'un "complexe de silicium organique de nouvelle génération", en écho aux « *silicium organiques de première et seconde génération* » évoqués en préambule, ou encore la conception et la mise au point d'un "complexe hybride de silicium organique".

L'invention a donc pour objet une composition, de préférence sous forme liquide, avantageusement sous forme de solution, comprenant :
a) un premier organo-silanol de formule générale (I) suivante :

   X-Si(OH)₃ (I)

   dans laquelle le radical X est un groupement alkyle en C₁-C₄, linéaire ou ramifié (avantageusement linéaire), de préférence en C₁-C₂, éventuellement substitué par au moins un groupement hydroxyle (de préférence par un groupement hydroxyle), de préférence X étant un groupement méthyle ;
b) un deuxième organo-silanol de formule générale (III) suivante :

   X-Si(OH)₂-O-CH(R₁)-CH(R₂)(R₃) (III)

   dans laquelle :
   - le radical X est un groupement alkyle en C₁-C₄, linéaire ou ramifié (avantageusement linéaire), de préférence en C₁-C₂, éventuellement substitué par au moins un groupement hydroxyle (de préférence par un groupement hydroxyle), de préférence X étant un groupement méthyle ;
   - R₁ est -H ou -CH₃;
   - R₂ est -H, -OH, ou -CH₃;
   - R₃ est -CH₃, -CH₂-CH₃, -CH₂OH, -CH(CH₃)OH, -CH₂-CH₂-CH₃, -CH₂-CH₂-CH₂-CH₃, -CH₂-CH₂-CH₂-CH₂-OH, ou -C(CH₃)(CH₃)-OH ; et,
c) un agent stabilisant/complexant d'organo-silanol(s), adapté pour permettre la formation d'un complexe moléculaire (stabilisant) avec au moins un organo-silanol via l'établissement d'au moins une liaison chimique faible, préférablement d'au moins une liaison hydrogène, avec ledit au moins un organo-silanol.

En effet, notamment en raison des effets techniques particulièrement avantageux i) et ii) mentionnés supra, la composition selon l'invention présente un silicium organique possédant notamment une très grande stabilité dans le temps, biodisponible et biologiquement actif (en particulier sur la peau).

Selon un mode de réalisation, le rapport molaire entre ledit premier organo-silanol et ledit deuxième organo-silanol est compris entre 100/1 et 100/20, de préférence entre 100/10 et 100/15.

Selon un mode de réalisation préféré, la composition selon l'invention est une solution hydroglycolique, à savoir comprenant un mélange d'eau et d'au moins un solvant glycolique. En effet, cette solution hydroglycolique représente un environnement optimal pour permettre la transformation d'une fraction de l'organo-silanol de formule générale (I) en organo-silanol de formule générale (III), via une réaction de monoalcoxylation entre le solvant glycolique retenu et l'un des groupements hydroxyle (portés par l'atome de silicium) de l'organo-silanol de formule générale (I), conduisant *in situ* à la formation spontanée, chimiquement équilibrée avec de l'organo-silanol de formule générale (I) coexistant, d'un nouvel organo-silanol.

En effet et si l'on prend l'exemple du méthylsilanetriol, la demanderesse a découvert, de manière inattendue, lors d'une analyse par spectroscopie de RMN ²⁹Si d'un méthylsilanetriol stabilisé introduit en solution hydroglycolique (analyse menée à des fins de s'assurer notamment de sa qualité majoritairement monomérique) :
- la création d'une liaison covalente de type -Si-O-C- issue d'une réaction de monoalcoxylation entre le glycol retenu (en l'espèce, il fut primairement choisi le 1,3-propanediol) et l'un des hydroxyles portés par l'atome de silicium du méthylsilanetriol (ce dernier pourtant attendu par la demanderesse exclusivement sous forme de complexe stabilisé par des liaisons hydrogènes), et ce pour conduire *in situ* à la formation spontanée, chimiquement équilibrée avec du méthylsilanetriol coexistant, d'une nouvelle espèce chimique ;
- la formation résultante, au titre de cette nouvelle espèce chimique siliciée, du composé "(3-hydroxypropoxy)-(méthyl)silanediol" dans le cas des méthysilanetriol et 1,3-propanediol susvisés.

A propos de ce nouveau composé "(3-hydroxypropoxy)-(méthyl)silanediol", la demanderesse souligne qu'il est ainsi nommé par le système de dénomination standardisée des composés chimiques (nomenclature IUPAC). Structuralement, il est représenté par la formule chimique semi-développée suivante : CH₃-Si(OH)₂-O-CH₂-CH₂-CH₂-OH. Enfin, son identification en solution hydroglycolique a résulté de l'analyse spectrale suivante : d'une part, ledit nouveau composé porteur de ladite nouvelle liaison covalente -Si-O-C- se caractérise, sur le spectre de contrôle en RMN ²⁹Si de la solution hydroglycolique, par la présence d'un singulet, net et surtout inédit, avec un déplacement chimique de moins 39,1 ppm (δ = - 39,1 ppm), alors que sur le même spectre figure, de manière attendue par la demanderesse, un autre singulet, d'intensité plus élevée, avec un déplacement chimique distinct de moins 38,6 ppm (δ = - 38,6 ppm), correspondant, lui, à un méthylsilanetriol « traditionnellement » stabilisé par des interactions faibles (en l'espèce par des liaisons hydrogènes) avec l'agent stabilisant/complexant utilisé, en l'espèce la molécule adénosine (ou xyloxyladenine) [cf. test 1 ci-après et mode de préparation]. D'autre part et au regard de l'enseignement fourni par des données spectrales de l'état de la technique (Sugahara Y. et al., J. Non-Cryst. Solids, 1994, vol.167, pp.21-28), la différence de déplacement chimique entre les deux singulets (Δδ = 0,5 ppm) est attribuable à la formation de la nouvelle liaison covalente -Si-O-C-,

En outre, un tel solvant glycolique présente l'avantage d'être miscible à l'eau, ce qui représente une réponse satisfaisante à l'objectif de produire une version alternative (et améliorée) de silicium organique stabilisé y compris avec des agents stabilisants/complexants affichant des caractéristiques plutôt contraires : hydrosolubilité insuffisante, etc. Ceci représente un avantage significatif dans la mesure où, tel qu'indiqué en préambule de la présente demande, un des objectifs de la présente invention consiste à développer, dans un solvant polaire (eau/solvant glycolique, par exemple), une génération alternative et améliorée d'un silicium organique, possédant notamment une très grande stabilité dans le temps, étant biodisponible et biologiquement actif (en particulier sur la peau).

De surcroît, un tel comportement réactionnel du silicium organique, en particulier de l'organo-silanol de formule générale (I), en solution hydroglycolique dans le respect de conditions maîtrisées (déterminables par l'homme du métier, sans difficulté excessive, sur la base de l'enseignement de la présente demande de brevet, complétée, le cas échéant, par ses connaissances générales), s'est avéré particulièrement avantageux et prometteur dans le contexte de la présente invention, notamment au regard des propriétés avantageuses exposées par un tel silicium organique en solution hydroglycolique, illustrées par :
- une capacité à afficher une constante de perméabilité améliorée, en accord avec un silicium biodisponible et biologiquement actif jusque dans les couches les plus profondes de l'épiderme [cf. test 2 ci-après] ;
- une capacité à présenter une très grande stabilité dans le temps en accord avec l'un des objectifs énoncés ci-avant [cf. test 3 ci-après] ;
- une capacité à présenter biologiquement une activité cytostimulante [cf. test 4 ci-après] ;
- une capacité à protéger des cellules présentes dans l'épiderme et le derme d'un état cytotoxique et oxydatif induit par une situation de stress [cf. test 5 ci-après] ;
- une capacité à limiter la production d'un médiateur lipidique impliqué dans la réponse de la peau à une situation de stress [cf. test 6 ci-après].

Un autre intérêt est que certains glycols, tels le propylène glycol dans l'industrie cosmétique, sont désormais exploités comme solvants organiques et humectants mais aussi pour leurs vertus antimicrobiennes qui leur confèrent un statut de suppléant à certains agents conservateurs réglementés mais à l'innocuité remise en cause ces dernières années (parabènes, etc.).

Selon un mode de réalisation préféré de l'invention, ledit au moins un solvant glycolique répond à la formule générale (II) suivante :

HO-CH(R₁)-CH(R₂)(R₃) (II)

dans laquelle :
- R₁ est -H ou -CH₃ ;
- R₂ est -H, -OH, ou -CH₃;
- R₃ est -CH₃, -CH₂-CH₃, -CH₂OH, -CH(CH₃)OH, -CH₂-CH₂-CH₃, -CH₂-CH₂-CH₂-CH₃, -CH₂-CH₂-CH₂-CH₂-OH, ou -C(CH₃)(CH₃)-OH.

Selon un mode de réalisation particulier, ledit au moins un solvant glycolique répond à la formule générale (IV) suivante :

HO-CH₂-(CH)ₙ(R)-OH (IV)

dans laquelle :
- le radical R est un atome d'hydrogène ou un groupement alkyle en C₁-C₄, notamment en C₁-C₂, de préférence un atome d'hydrogène; et
- n est un entier naturel compris entre 1 et 4, de préférence entre 1 et 3, de manière préférée n étant 2.

Selon un autre mode de réalisation particulier, ledit au moins un solvant glycolique est choisi parmi le 1,2-propanediol, le 1,3-propanediol, le 1,2-butanediol, le 1,3-butanediol, le 2-méthyl-1,3-propanediol, le 1,2-3-propanetriol, le 1,2-hexanediol, le 1,6-hexanediol, le 1,2-dihydroxypentane, l'hexylène glycol, le 3-méthylbutane-1,2-diol, et leurs mélanges, de préférence parmi les 1,2-propanediol, 1,3-propanediol, 1,3-butanediol, 2-méthyl-1,3-propanediol, 1,2,3-propanetriol, et leurs mélanges ; de manière préférée ledit au moins un solvant glycolique étant le 1,3-propanediol.

De préférence, la composition selon l'invention est telle que:
i) le rapport massique eau/solvant glycolique est compris (selon la nature du glycol) entre 98/2 et 20/80, de préférence entre 80/20 et 20/80, avantageusement entre 80/20 et 50/50, et/ou
ii) le pH de ladite composition est compris entre environ 4 et environ 6 (par exemple entre 4 et 6) ;
de manière préférée, ladite composition présentant les caractéristiques i) et ii).

Les travaux de la demanderesse ont permis de mettre en évidence le fait que les susdits paramètres/conditions opératoires i) et ii) permettaient d'obtenir une composition hydroglycolique, à savoir un mélange d'eau et d'au moins un solvant glycolique, de préférence une solution hydroglycolique, présentant des propriétés optimales en termes de stabilité, de biodisponibilité et d'activité biologique du silicium organique qu'elle renferme. Avantageusement, la composition selon l'invention est telle que ledit agent stabilisant/complexant d'organo-silanol(s) est une substance biologiquement active (par exemple une substance physiologiquement active), préférablement choisie parmi l'adénosine, l'acide glyccyrrhétinique et/ou ses sels, la glycyrrhizine et/ou ses sels, l'acide lactobionique et/ou ses sels, l'acide alginique et/ou ses sels, l'acide hyaluronique et/ou ses sels, le lactose, le tréhalose, le 6-déoxy-L-mannopyranose et/ou ses sels, l'acide théophylline acétique, l'acide ascorbique, l'acide lactique, l'acide salicylique et/ou ses sels, l'acide pyrrolidone carboxylique et/ou ses sels, l'arginine, la sérine, la lysine et/ou leurs sels, la méthionine et/ou ses sels, l'acétyl-méthionine, la thréonine, l'hydroxyproline, la N-acétyl-tyrosine, l'acide aspartique, l'acide glutamique et/ou leurs sels, l'alcool oléique, le panthénol, la caféine, la pectine, l'acéfylline, le sulfate de chondroïtine et/ou leurs sels, la perle hydrolysée, un hydrolysat de protéines d'origine animale ou végétale, par exemple un hydrolysat de collagène d'origine marine tel qu'un hydrolysat de collagène de peau(x) de poisson(s), et leurs mélanges, avantageusement choisie parmi l'acide alginique et/ou ses sels, le 6-déoxy-L-mannopyranose et/ou ses sels, l'acide hyaluronique et/ou ses sels, l'acide ascorbique, l'hydroxyproline, l'acide théophylline acétique, l'acide salicylique et/ou ses sels, l'adénosine, la caféine, et leurs mélanges. De manière particulièrement préférée, ladite substance biologiquement active est l'acide alginique et/ou ses sels, ou l'acide hyaluronique et/ou ses sels, ou un mélange d'acide alginique (et/ou de ses sels) et d'acide hyaluronique (et/ou de ses sels). A propos des substances biologiquement actives susnommées, elles peuvent être notamment définies par un numéro d'enregistrement auprès d'une banque de données, telle la banque de données de la division "Chemical Abstracts Service (CAS)". Ainsi comme exemple d'acide alginique et/ou ses sels, on peut citer l'acide alginique (n° CAS : 9005-32-7) ainsi que les sels de sodium, potassium et calcium de l'acide alginique (n° CAS respectifs : 9005-38-3, 9005-36-1, 9005-35-0). Comme exemple d'acide hyaluronique et/ou ses sels, on peut citer un acide hyaluronique de haut poids moléculaire (> à 1.400 kDa) et un acide hyaluronique de bas poids moléculaire (< à 700 kDa), en particulier sous forme de sels de sodium.

Le fait que l'agent stabilisant/complexant d'organo-silanol(s) soit une substance biologiquement active présente le double avantage de permettre la stabilisation des susdits premier et deuxième organo-silanols, notamment via la formation du complexe ternaire de schéma susvisé, ce qui correspond à sa « fonction première », mais également d'avoir une ou plusieurs action(s) biologique(s) (par exemple physiologique(s)) sur l'organisme.

L'invention concerne également une composition pharmaceutique, un médicament à usage humain ou vétérinaire, un dispositif médical tel qu'une solution injectable, un complément alimentaire à usage humain ou animal, une composition cosmétique, une composition dermo-cosmétique, comprenant (ou consistant essentiellement en) une composition selon l'invention. Un autre objet de l'invention concerne une composition selon l'invention pour son utilisation en tant que médicament à usage humain ou vétérinaire (de préférence à usage humain). L'invention concerne également une composition selon l'invention pour son utilisation dans la prévention et/ou la limitation des dégradations cutanées liées au vieillissement de la peau, en particulier liées à un stress oxydatif (radiations ultraviolettes, pollution atmosphérique, contact avec des xénobiotiques chimiques, etc.) générant des radicaux libres ou des espèces réactives de l'oxygène, pour relancer l'activité cellulaire épidermique, dermique et hypodermique, et/ou comme agent stimulant sur l'expression de collagènes fibrillaires et protéoglycanes constitutifs de la jonction dermo-épidermique (à savoir pour stimuler l'expression de collagènes fibrillaires et protéoglycanes constitutifs de la jonction dermo-épidermique), et/ou comme agent adapté pour réduire la taille et/ou la visibilité des pores visibles ("noticeable pores", en langue anglaise) de la peau liés à l'âge. L'invention concerne donc également l'utilisation d'une composition selon l'invention, telle que définie au sein de la présente demande de brevet, pour réduire la taille et/ou la visibilité des pores visibles de la peau liés à l'âge. Selon un mode de réalisation particulier de l'invention vis-à-vis d'une telle action sur les pores de la peau, ledit agent stabilisant/complexant d'organo-silanol(s) adapté pour permettre la formation d'un complexe moléculaire avec au moins un organo-silanol est l'adénosine.

L'invention a également pour objet un organo-silanol de formule générale (III) suivante :

X-Si(OH)₂-O-CH(R₁)-CH(R₂)(R₃) (III)

dans laquelle :
- le radical X est un groupement alkyle en C₁-C₄, linéaire ou ramifié (avantageusement linéaire), de préférence en C₁-C₂, éventuellement substitué par au moins un groupement hydroxyle (de préférence par un groupement hydroxyle), de préférence X étant un groupement méthyle ;
- R₁ est -H ou -CH₃ ;
- R₂ est -H, -OH, ou -CH₃;
- R₃ est -CH₃, -CH₂-CH₃, -CH₂OH, -CH(CH₃)OH, -CH₂-CH₂-CH₃, -CH₂-CH₂-CH₂-CH₃, -CH₂-CH₂-CH₂-CH₂-OH, ou -C(CH₃)(CH₃)-OH.

Selon un mode de réalisation particulier, ledit organo-silanol de formule générale (III) répond à la formule générale (V) suivante :

X-Si(OH)₂-O-CH₂-(CH)ₙ(R)-OH (V)

dans laquelle :
- le radical X est un groupement alkyle en C₁-C₄, linéaire ou ramifié (avantageusement linéaire), de préférence en C₁-C₂, éventuellement substitué par au moins un groupement hydroxyle (de préférence par un groupement hydroxyle), de préférence X étant un groupement méthyle ;
- le radical R est un atome d'hydrogène ou un groupement alkyle en C₁-C₄, notamment en C₁-C₂, de préférence un atome d'hydrogène; et
- n est un entier naturel compris entre 1 et 4, de préférence entre 1 et 3, de manière préférée n étant 2.

Selon un autre mode de réalisation particulier, ledit organo-silanol de formule générale (III) est le produit de réaction d'une monoalcoxylation entre l'un des groupements hydroxyle portés par l'atome de silicium de l'organo-silanol de formule générale (I), tel que défini supra, et un solvant glycolique choisi parmi le 1,2-propanediol, le 1,3-propanediol, le 1,2-butanediol, le 1,3-butanediol, le 2-méthyl-1,3-propanediol, le 1,2-3-propanetriol, le 1,2-hexanediol, le 1,6-hexanediol, le 1,2-dihydroxypentane, l'hexylène glycol, le 3-méthylbutane-1,2-diol, et leurs mélanges, de préférence parmi les 1,2-propanediol, 1,3-propanediol, 1,3-butanediol, 2-méthyl-1,3-propanediol, 1,2,3-propanetriol, et leurs mélanges.

De préférence, le susdit composé organo-silanol de formule générale (III) est tel que X est un groupement méthyle, R₁ est un atome d'hydrogène, R₂ est un atome d'hydrogène, et R₃ est - CH₂-OH.

Ledit organo-silanol est le (3-hydroxypropoxy)-(méthyl)silanediol. Tel qu'indiqué précédemment, le composé affiche en spectroscopie de RMN ²⁹Si (liquide) un déplacement chimique à δ = - 39,1 ppm.

Un autre objet de l'invention concerne un procédé de préparation d'un organo-silanol de formule générale (III) susmentionnée, comprenant les étapes suivantes :
a) obtenir un organo-silanol de formule générale (I) suivante :

   X-Si(OH)₃ (I)

   dans laquelle le radical X est un groupement alkyle en C₁-C₄, linéaire ou ramifié (avantageusement linéaire), de préférence en C₁-C₂, éventuellement substitué par au moins un groupement hydroxyle (de préférence par un groupement hydroxyle), de préférence X étant un groupement méthyle ;
b) mettre en contact ledit organo-silanol de formule générale (I) avec au moins un solvant glycolique répondant à la formule générale (II) suivante :

   HO-CH(R₁)-CH(R₂)(R₃) (II)

   dans laquelle :
   - R₁ est -H ou -CH₃ ;
   - R₂ est -H, -OH, ou -CH₃;
   - R₃ est -CH₃, -CH₂-CH₃, -CH₂OH, -CH(CH₃)OH, -CH₂-CH₂-CH₃, -CH₂-CH₂-CH₂-CH₃, -CH₂-CH₂-CH₂-CH₂-OH, ou -C(CH₃)(CH₃)-OH-
   durant un laps de temps suffisant pour obtenir, via une réaction de monoalcoxylation, la formation du susdit organo-silanol de formule générale (III).

Ce procédé de préparation est notamment illustré par le test 1 ci-après tel qu'indiqué supra, mais également par le test 7 ci-après qui concerne, de manière distincte au test 1, d'autres d'organo-silanols de formule générale (I) que le méthylsilanetriol et/ou d'autres agents stabilisants/complexants que l'adénosine et/ou d'autres solvants glycoliques de formule générale (II) que le 1,3-propanediol. Pour toutes ces autres illustrations d'une composition hydroglycolique selon l'invention, la présence des susdits organo-silanols de formule générale (I) et de formule générale (III) a été confirmée en spectroscopie de RMN ²⁹Si (liquide) par l'observation, sur chaque spectre de contrôle, de deux singulets d'intensité distincte mais de déplacements chimiques (δ) très voisins des valeurs indicatives de moins 39,1 ppm (δ = - 39,1 ppm) et moins 38,6 ppm (δ = - 38,6 ppm) primairement et spécifiquement relevées pour le silicium organique en solution hydroglycolique objet du test 1, la différence de déplacement chimique (Δδ) entre les deux singulets étant dépendante de la nature des réactifs silylés conduisant à l'organo-silanol de formule générale (I) et de la nature du solvant glycolique de formule (II) tous deux engagés dans la formation de la nouvelle liaison covalente -Si-O-C- [cf. test 7 ci-après].

L'invention concerne également l'utilisation d'un organo-silanol selon la formule générale (III), pour accroître la stabilité d'une composition/d'un système comprenant :
- un organo-silanol de formule générale (I) suivante :

   X-Si(OH)₃ (I)

   dans laquelle le radical X est un groupement alkyle en C₁-C₄, linéaire ou ramifié (avantageusement linéaire), de préférence en C₁-C₂, éventuellement substitué par au moins un groupement hydroxyle (de préférence par un groupement hydroxyle), de préférence X étant un groupement méthyle ; et
- un agent stabilisant d'organo-silanol(s) tel que défini supra.

L'invention s'étend également à une composition, préférentiellement cosmétique ou dermocosmétique destinée à prévenir et/ou réparer les dégradations cutanées liées au vieillissement. Cette composition comprend, en association avec tout excipient physiologiquement compatible avec la peau, à titre d'ingrédient actif principal, une composition selon l'invention, et en particulier une composition hydroglycolique selon l'invention, de préférence sous forme de solution hydroglycolique, telle que définie ci-dessus. Ladite composition, préférentiellement cosmétique ou dermocosmétique, est adaptée à une administration par voie topique cutanée, et présentée sous toutes les formes normalement utilisées pour une telle administration. A titre indicatif et non limitatif, les compositions peuvent se présenter sous la forme de suspensions, lotions, crèmes, gels aqueux ou hydroalcooliques, poudres et émulsions multiples pouvant être éventuellement des microémulsions ou des nanoémulsions, etc. Ladite composition, préférentiellement cosmétique ou dermocosmétique, peut contenir comme excipent compatible avec la peau au moins un excipient connu de l'homme du métier et acceptable dans les domaines cosmétique ou dermocosmétique, choisi parmi les huiles, les cires, les élastomères de silicone, les tensioactifs, les co-tensioactifs, les épaississants et/ou gélifiants, les humectants, les émollients, les filtres solaires organiques ou inorganiques, les photostabilisants, les conservateurs à l'exception des conservateurs donneurs d'aldéhydes, les colorants, les agents matifiants, les corps gras, les pigments, les agents tenseurs, les séquestrants, les parfums, etc., et leurs mélanges. Ladite composition, préférentiellement cosmétique ou dermocosmétique, peut en outre comprendre un ou plusieurs ingrédients actifs additionnels, choisi(s) - sans que cette liste ne soit limitative - parmi les agents déglycants, les agents stimulant la synthèse de collagène ou d'élastine ou prévenant leur dégradation, les agents stimulant la synthèse de glycosaminoglycanes ou de protéoglycanes ou prévenant leur dégradation, les agents augmentant la prolifération cellulaire, les agents dépigmentants ou pro-pigmentants, les agents antioxydants ou anti-radicalaires ou anti-pollution, les agents hydratants, les agents stimulant la lipolyse, les agents drainants ou détoxifiants, les agents anti-inflammatoires, les agents accélérateurs de pénétration, les agents desquamants, les agents apaisants et/ou anti-irritants, les agents astringents, les agents agissant sur la microcirculation, etc., et leurs mélanges.

Par ailleurs, l'invention a également pour objet l'utilisation de la composition selon l'invention, et en particulier une composition hydroglycolique selon l'invention, de préférence sous forme de solution hydroglycolique comme ingrédient actif cosmétique ou dermocosmétique, ou pour la fabrication de préparations ou de compléments alimentaires, diététiques, cosmétiques ou pharmaceutiques à usage humain ou animal. Avantageusement, la composition selon l'invention dans les compositions, préparations, dispositifs médicaux ou compléments susvisés est comprise entre 1 % et 15 % (par exemple entre 1% et 10%) en poids par rapport au poids total de la composition, préparation, dispositif médical ou complément susvisé(e), de préférence entre 3 % et 8 % (par exemple entre 3% et 7%) en poids.

### Définitions

Par « silicium organique », il faut comprendre un ou plusieurs composé(s) organo-silanol(s). Par « agent stabilisant/complexant d'organo-silanol(s) » (parfois abrégé au sein de la présente demande en « agent stabilisant/complexant » ou simplement dénommé «agent stabilisant d'organo-silanol(s) », à des fins de concision), il faut comprendre une molécule, un composé, une substance ou une entité adaptés pour permettre la formation d'un complexe moléculaire avec au moins un organo-silanol via l'établissement d'au moins une liaison chimique faible, préférablement d'au moins une liaison hydrogène, avec ledit au moins un organo-silanol. Par « liaisons hydrogènes », il faut comprendre les liaisons hydrogènes telles que définies par l'Union Internationale de Chimie Pure et Appliquée dans ses recommandations (Arunan E. et al., Pure Appl. Chem., 2011, vol.83, pp. 1637-1641).

Par « stable» ou « stabilité dans le temps », il faut comprendre une stabilité du silicium organique en solution d'au moins, et sans la moindre opalescence observée, de 9 mois.

Par « biodisponibilité », il faut comprendre la capacité d'une molécule à être absorbée et être distribuée dans l'organisme. Par exemple, dans le cas d'une administration par voie topique cutanée, la biodisponibilité s'entend d'une capacité à pénétrer le stratum corneum jusqu'aux couches les plus profondes de la peau (épiderme, derme, hypoderme).

« Biologiquement actif » ou « actif biologiquement » désigne la propriété, pour une molécule, une substance, un composé ou une entité, de présenter une action/activité biologique, c'est-à-dire que son introduction dans le vivant va entraîner une réaction de celui-ci (qui peut être bénéfique ou néfaste). Il va sans dire que, dans le contexte de la présente invention, l'action/activité biologique recherchée est de nature « bénéfique ».

### Description détaillée

La description détaillée ci-après a pour but d'exposer l'invention de manière suffisamment claire et complète, notamment à l'aide d'exemples, mais ne doit en aucun cas être regardée comme limitant l'étendue de la protection aux modes de réalisation particuliers et aux exemples présentés ci-après.

### Exemples

### Test 1 : procédé de préparation d'un silicium organique en solution hydroglycolique selon l'invention (à savoir le susnommé "complexe ternaire représenté par le schéma OS(I)---AS-OS(III)") impliquant les substances méthylsilanetriol et (3-hydroxypropoxy)-(méthyl)silanediol ainsi que l'adénosine comme agent stabilisant/complexant

Dans un premier temps, 8,55 g d'adénosine (32 mmoles) sont suspendus dans environ 320 g d'eau. Ensuite, 6,92 g d'une solution commerciale de méthylsiliconate de sodium (équivalent à 32 mmoles de méthylsilanetriol), préalablement dilués dans env. 40 ml d'eau, sont ajoutés à la suspension précédente. Le pH résultant est ajusté à 4,95 à l'aide de quelques gouttes d'HCl 1N. 500 g de 1,3-propanediol sont alors introduits sous agitation, à température ambiante. La masse de solution est finalement ajustée à 1kg par addition d'eau. La solution résultante de complexe (dans un rapport massique eau/1,3-propanediol 50/50) est finalement ajustée à un pH de 5 à l'aide de quelques gouttes d'HCl 1N. On obtient une solution limpide, incolore et inodore.

### Test 2 : profil de pénétration cutanée pour le silicium organique en solution hydroglycolique

Principe : l'épiderme de la peau est composé de quatre couches allant de la plus superficielle (le "stratum corneum") à la plus profonde (la "couche basale"). Pour une entité organique structuralement définie, il est ainsi possible de déterminer son coefficient de partage octanol/tampon ("Log P") permettant :
- la détermination de la constante de perméabilité cutanée ("Kp") à partir de l'équation modèle dite de "Potts & Guy" (selon l'article « Predicting skin permeability ») (Potts R.O. & Guy R.H., Pharm. Res., 1992, vol.9, pp. 663-669) ;
- et ainsi d'exprimer pour l'entité visée sa capacité à traverser la barrière cutanée épidermique (Arct J. et al., SOFW J., 2003, vol. 129, pp. 2-9).

Dans le cas de la présente invention, une détermination théorique du Log P a été réalisée à l'aide du logiciel de prédiction "Chemdraw ^{®}" commercialisé par la société « Cambridge soft », pour les deux cas suivants :
- cas 1 : méthylsilanetriol associé et stabilisé, en solution exclusivement aqueuse, par un agent stabilisant/complexant (liaisons faibles)
- cas 2 : silicium organique en solution hydroglycolique (eau/glycol : 50/50) selon l'invention (à savoir le susnommé "complexe ternaire représenté par le schéma OS(I)---AS---OS(III)") impliquant les substances méthylsilanetriol, (3-hydroxypropoxy)-(méthyl)silanediol, et un agent stabilisant/complexant. Les résultats sont rassemblés dans le tableau 1 suivant :

**Tableau 1**

| **Silicium organique** | **Log P** | **Kp (cm.h⁻¹).10⁻⁵** |
|---|---|---|
| Cas 1 | -1,30 | 5,8 |
| Cas 2 (selon l'invention) | -0,71 | 6,7 |

Etant admis que le flux (soit la quantité de soluté qui passe dans la peau par unité de surface) est proportionnel à "Kp" et que, plus le "Kp" est élevé plus le produit pénètre, les résultats ci-dessus témoignent d'une constante de perméabilité supérieure pour la composition selon l'invention. Il est donc prédit, pour une telle composition ("complexe ternaire"), une capacité supérieure à traverser la barrière cutanée épidermique et donc une biodisponibilité supérieure à celle, par exemple d'un méthylsilanetriol appartenant à l'état de la technique et stabilisé par des liaisons faibles (liaisons hydrogènes).

### Test 3 : mise en évidence de la stabilité dans le temps d'un silicium organique en solution hydroglycolique

Principe : la stabilité de complexes de méthylsilanetriol avec divers éléments métalliques de la classification périodique, en milieu acide, est connue et a été étudiée (Vevere I. et al., Latvijas K 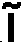 mijas 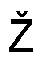 urnãls, 1996, vol.1, pp.70-73, et références citées). Elle vise à constater l'apparition d'espèces insolubles en solution aqueuse, qui, sous un faisceau lumineux incident et réfléchi, s'expriment visuellement par une opalescence bleue de la solution.

Il a donc été mené une même étude de stabilité que celle de l'état de la technique, pour les deux cas suivants :
- cas 1 : complexe de méthylsilanetriol associé et stabilisé, en solution exclusivement aqueuse, par des liaisons faibles (liaisons hydrogènes) avec l'acide ascorbique
- cas 2 : silicium organique en solution hydroglycolique (eau/glycol : 50/50) selon l'invention (à savoir le susnommé "complexe ternaire représenté par le schéma OS(I)---AS---OS(III)") impliquant les substances méthylsilanetriol et (3-hydroxypropoxy)-(méthyl)silanediol ainsi que le même agent stabilisant/complexant du cas 1

Les résultats sont rassemblés dans le tableau 2 suivant :

**Tableau 2**

| | **Stabilité maximale** | |
|---|---|---|
| **Silicium organique** | **pH** | **T (jours)** |
| Cas 1 | 4,0-5,0 | 110 |
| Cas 2 (selon l'invention) | 5,0-6,0 | 551 |

Les résultats soulignent une stabilité largement supérieure d'un silicium organique en solution hydroglycolique selon l'invention sur celle d'un même silicium organique mais dépourvu en solution d'un composé glycolique.

### Test 4 : mise en évidence de l'effet cytostimulant d'un silicium organique en solution hydroglycolique sur des épidermes reconstruits d'origine humaine

La mise en culture des EHR a consisté à les placer dans un milieu de croissance "MCDB 153" (fournisseur : SkinEthic^{®}) contenant 5mg/mL d'insuline, 1,5 mM de CaCl₂ et 25 mg/mL de gentamycine, pendant 24 heures à 37°C et 5% CO₂.

L'observation de la prolifération cellulaire est réalisée par la technique de l'immunomarquage (Gerdes et al., Int. J. Cancer (1983), vol. 31, pp. 13-20) en utilisant le marqueur de prolifération cellulaire "Ki67", pour les trois configurations suivantes (après un dépôt biquotidien de 100 µl sur les EHR) :
- cas 1 : solution tampon de PBS (contrôle)
- cas 2 : complexe de méthylsilanetriol associé et stabilisé, en solution exclusivement aqueuse, par des liaisons faibles (hydrogènes) avec l'acide alginique
- cas 3 : silicium organique en solution hydroglycolique (eau/glycol : 50/50) selon l'invention (à savoir le susnommé "complexe ternaire représenté par le schéma OS(I)---AS---OS(III)") impliquant les substances méthylsilanetriol et (3-hydroxypropoxy)-(méthyl)silanediol ainsi que le même agent stabilisant/complexant du cas 2

Les résultats obtenus sont rassemblés dans le tableau 3 ci-après :

**Tableau 3**

| **Silicium organique** | **Concentration (%)** | **% cellules exprimant Ki67 / nombre de cellules totales** (% par rapport au contrôle) |
|---|---|---|
| Cas 1 (contrôle) | - | - |
| Cas 2 | 5 | + 35 ± 0,5 |
| Cas 3 (selon l'invention) | 5 | + 58 ± 1 |

Les résultats soulignent une cytostimulation potentialisée pour le silicium organique en solution hydroglycolique selon l'invention, supérieure à celle d'un même silicium organique mais dépourvu en solution d'un composé glycolique.

### Test 5 : détermination et mise en évidence de la capacité d'un silicium organique en solution hydroglycolique selon l'invention à limiter la synthèse endogène d'un agent de signalisation intracellulaire, le monoxyde d'azote (NO)

En préambule, il est indiqué que le monoxyde d'azote est une espèce radicalaire libre impliquée dans les mécanismes de vieillissement de la peau (Ahsanuddin S. et al., AIMS Molecular Sciences, 2016, vol.3, pp.187-195). Ensuite expérimentalement, le test a été réalisé sur des macrophages murins de lignée cellulaire "RAW 264.7". Ces macrophages ont été cultivés dans un milieu de culture complet "DMEM" (avec 4,5 g/l de glucose et 10% de sérum de veau foetal (SVF)), puis maintenus dans une atmosphère à 37°C et 5% de CO₂. A J-1, les macrophages ont été ensemencés en plaques de 24 puits à raison de 65789 cellules/cm². A J0 + 2 heures, les macrophages sont ensuite soumis à un stress généré par l'application de lipopolysaccharides (LPS), induisant la production de monoxyde d'azote (LPS à 10 ng/ml), puis sont incubés à 37°C et 5 % de CO₂ pendant 22 heures supplémentaires toujours en présence des traitements. A J+1, les surnageants de culture ont été récoltés afin de réaliser une quantification de monoxyde d'azote/nitrites (Griess Reagent Kit for nitrite quantitation, Invitrogen, reference G7921) et une analyse statistique en triplicate ou quadriplicate (selon le complexe), à l'absorbance de 540 nm, pour les cinq configurations suivantes :
- cas 1 : contrôle avec stress LPS (abréviation "LPS-stress")
- cas 2 : complexe de méthylsilanetriol associé et stabilisé, en solution exclusivement aqueuse, par des liaisons faibles (hydrogènes) avec l'hydroxyproline
- cas 3 : silicium organique en solution hydroglycolique (eau/glycol : 50/50) selon l'invention (à savoir le susnommé "complexe ternaire représenté par le schéma OS(I)---AS---OS(III)") impliquant les substances méthylsilanetriol et (3-hydroxypropoxy)-(méthyl)silanediol ainsi que le même agent stabilisant/complexant du cas 2
- cas 4 : complexe de méthylsilanetriol associé et stabilisé, en solution exclusivement aqueuse, par des liaisons faibles (hydrogènes) avec le 6-déoxy-L-mannopyranose
- cas 5 : silicium organique en solution hydroglycolique (eau/glycol : 50/50) selon l'invention (à savoir le susnommé "complexe ternaire représenté par le schéma OS(I)---AS---OS(III)") impliquant les substances méthylsilanetriol et (3-hydroxypropoxy)-(méthyl)silanediol ainsi que le même agent stabilisant/complexant du cas 4

Identiquement aux tests 2 à 4 susmentionnés, le présent test permet une comparaison, pour une même concentration, d'un comportement biologique *in vitro* entre un complexe de méthylsilanetriol associé et stabilisé, en solution exclusivement aqueuse et un même complexe de méthylsilanetriol associé et stabilisé mais en solution hydroglycolique.

Les résultats sont rassemblés dans le tableau 4 ci-après, notamment exprimés en % d'inhibition de la sécrétion de monoxyde d'azote par rapport au contrôle non traité.

**Tableau 4**

| Composé | Quantité de NO sécrétée (% LPS) | % d'inhibition de la sécrétion de NO |
|---|---|---|
| Cas 1 - Contrôle ("LPS-stress") | 100 | N/A |
| Cas 2 - "LPS-stress" + silicium organique à la conc. de 0,5% | 75 | - 25 % |
| Cas 3 - "LPS-stress" + silicium organique à la conc. de 0,5% | 55 | - 45 % |
| Cas 4 - "LPS-stress" + silicium organique à la conc. de 0,25% | 74 | - 26 % |
| Cas 5 - "LPS-stress" + silicium organique à la conc. de 0,25% | 62 | - 38 % |

Les résultats soulignent une inhibition de la production de monoxyde d'azote induite par le stress LPS potentialisée pour le silicium organique en solution hydroglycolique selon l'invention, supérieure à celle d'un même silicium organique mais dépourvu en solution d'un composé glycolique.

### Test 6 : détermination et mise en évidence de la capacité d'un silicium organique en solution hydroglycolique selon l'invention à limiter la production d'un médiateur lipidique, la prostaglandine E₂ (PGE2),

En préambule, il est indiqué que la prostaglandine E₂ (PGE2) est un marqueur notamment désigné pour étudier les effets du vieillissement sur la production de collagène de type I (Shim J.H, Int. J. Mol. Sc., 2019, vol.20, 5555, pp.1-12). Ensuite expérimentalement, le test a été réalisé dans les mêmes conditions que le test 5 ci-dessus, à savoir une même lignée de macrophages et une même mise en culture, à l'exception de l'étape suivante : à J+1, les surnageants de culture ont été récoltés afin de réaliser une quantification de PGE2 sécrétée (Prostaglandin E2 Parameter Assay Kit, référence KGE004B, R&D System) et une analyse statistique en triplicate, à l'absorbance de 450 nm, les trois configurations testées étant :
- cas 1 : contrôle avec stress (LPS-stress)
- cas 2 : complexe de méthylsilanetriol associé et stabilisé, en solution exclusivement aqueuse, par des liaisons faibles (hydrogènes) avec l'acide hyaluronique (< à 700 kDa)
- cas 3 : silicium organique en solution hydroglycolique (eau/glycol : 50/50) selon l'invention (à savoir le susnommé "complexe ternaire représenté par le schéma OS(I)---AS---OS(III)") impliquant les substances méthylsilanetriol et (3-hydroxypropoxy)-(méthyl)silanediol ainsi que le même agent stabilisant/complexant du cas 2
- cas 4 : complexe de méthylsilanetriol associé et stabilisé, en solution exclusivement aqueuse, par des liaisons faibles (hydrogènes) avec le 6-déoxy-L-mannopyranose
- cas 5 : silicium organique en solution hydroglycolique (eau/glycol : 50/50) selon l'invention (à savoir le susnommé "complexe ternaire représenté par le schéma OS(I)---AS---OS(III)") impliquant les substances méthylsilanetriol et (3-hydroxypropoxy)-(méthyl)silanediol ainsi que le même agent stabilisant/complexant du cas 4.

Identiquement aux tests 2 à 5 susmentionnés, le présent test permet une comparaison, pour une même concentration, d'un comportement biologique *in vitro* entre un complexe de méthylsilanetriol associé et stabilisé, en solution exclusivement aqueuse et un même complexe de méthylsilanetriol associé et stabilisé mais en solution hydroglycolique.

Les résultats sont rassemblés dans le tableau 5 ci-après, notamment exprimés en % d'inhibition de la sécrétion de PGE2 par rapport au contrôle non traité.

**Tableau 5**

| Composé | Quantité de PGE₂ sécrétée (% LPS) | % d'inhibition de la sécrétion de PGE₂ |
|---|---|---|
| Cas 1 - Contrôle ("LPS-stress") | 100 | N/A |
| Cas 2 - "LPS-stress" + silicium organique à la conc. de 0,25% | 108 | N/A |
| Cas 3 - "LPS-stress" + silicium organique à la conc. de 0,25% | 74 | - 26 % |
| Cas 2 - "LPS-stress" + silicium organique à la conc. de 0,5% | 105 | N/A |
| Cas 3 - "LPS-stress" + silicium organique à la conc. de 0,5% | 66 | - 34 % |
| Cas 2 - "LPS-stress" + silicium organique à la conc. de 1% | 99 | - 1% |
| Cas 3 - "LPS-stress" + silicium organique à la conc. de 1% | 63 | - 27 % |
| Cas 4 - "LPS-stress" + silicium organique à la conc. de 0,25% | 99 | - 1% |
| Cas 5 - "LPS-stress" + silicium organique à la conc. de 0,25% | 78 | - 22 % |

Les résultats soulignent une inhibition de la production de PGE2 induite par le stress LPS, potentialisée pour le silicium organique en solution hydroglycolique selon l'invention, supérieure et dose-dépendante à celle d'un même silicium organique mais dépourvu en solution d'un composé glycolique.

### Test 7 : procédé de préparation d'un silicium organique en solution hydroglycolique selon l'invention (à savoir le susnommé "complexe ternaire représenté par le schéma OS(I)---AS-OS(III)") impliquant divers organo-silanols de formule générale (I), divers organo-silanols de formule générale (III), divers solvants glycoliques de formule générale (II), divers rapports massiques eau/solvant glycolique, enfin divers agents stabilisants/complexants

Expérimentalement, les procédés de préparation pour chacun des exemples illustratifs ci-après ont été réalisés selon la chronologie et les conditions opératoires décrites dans le test 1 ci-avant. Ils ont conduit, dans chacun des 10 exemples engendrés exposés dans le tableau 6 ci-après, à une solution limpide, incolore et inodore, qui a été l'objet ensuite d'une analyse en spectroscopie de RMN ²⁹Si à l'aide d'un spectromètre "Brucker Avance 500" et dans les conditions suivantes :
- température d'analyse : température ambiante (300 °K)
- temps de relaxation : 30 secondes
- nombre de scans : 6000
- durée d'analyse : 50 heures
- référence externe : D₂O (solvant deutéré)
- diamètre interne tube RMN : 10 mm
- noyau : ²⁹Si ; fréquence : 99.36 Mhz ; Pulse program : Zgig

**Tableau 6**

| **Réactif silylé** | **Solvant glycolique de formule (II)** | **Composition selon l'invention [complexe ternaire, stabilisé]** | | **Δδ (PPm)** |
|---|---|---|---|---|
| --- | **HO-CH(R₁)-CH(R₂)(R₃)** | | | |
| **Agent stabilisant [AS]** | --- | **OS(I)** -- **AS** -- **OS(III)** | | |
| | **Rapport massique H₂O/glycol** | | | |
| | | **OS(I) X-Si(OH)₃ [δ en ppm] Lot** | **OS(III) X-Si(OH)₂-O-CH(R₁)-CH(R₂)(R₃) [δ en ppm] Lot** | |
| méthylsiliconate de sodium | | X = -CH₃ | X = -CH₃ | 0,51 |
| | R₁ = H | | R₁ = H | |
| --- | R₂ = H | | R₂ = H | |
| acide hyaluronique | R₃ = -CH₂OH | | | |
| | --- | | R₃ = -CH₂OH | |
| | 50/50 | δ = -37,33 ppm | δ = - 37,84 ppm | |
| | | | | |
| | | E17384 | E17384 | |
| | | | | |
| méthylsiliconate de sodium | | X = -CH₃ | X = -CH₃ | 0,59 |
| | R₁ = H | | R₁ = H | |
| | R₂ = CH₃ | | R₂ = CH₃ | |
| --- | R₃ = -CH₂OH | | R₃ = -CH₂OH | |
| acide hyaluronique | --- | | | |
| | 50/50 | δ = -37,46 ppm | δ = - 38,05 ppm | |
| | | | | |
| | | E17390 | E17390 | |
| | | | | |
| méthylsiliconate de sodium | | X = -CH₃ | X = -CH₃ | 0,25 |
| | R₁ = H | | R₁ = H | |
| | R₂ = H | | R₂ = H | |
| --- | R₃ = -CH(CH₃)OH | | R₃ = -CH(CH₃)OH | |
| acide hyaluronique | --- | | | |
| | 50/50 | δ = -37,85 ppm | δ = - 38,10 ppm | |
| | | | | |
| | | E17385 | E17385 | |
| | | | | |
| méthylsiliconatede sodium | R₁ = H | | | 0,51 |
| --- | R₂ = H | X = -CH₃ | X = -CH₃ | |
| acide alginique | R₃ = -CH₂OH | | R₁ = H | |
| | --- | | R₂ = H | |
| | 50/50 | | R₃ = -CH₂OH | |
| | | δ = -37,34 ppm | δ = - 37,85 ppm | |
| | | --- | --- | |
| | | E17386 | E17386 | |
| | | | | |
| | R₁ = H | X = -CH₃ | X = -CH₃ | 0,52 |
| | | | R₁ = H | |
| méthysiliconate de sodium | R₂ = H | | R₂ = H | |
| --- | R₃ = -CH₂OH | | R₃ = -CH₂OH | |
| 6-déoxy-L-mannopyranose | --- | | δ = - 37,85 ppm | |
| | 50/50 | δ = -37,33 ppm | | |
| | | --- | --- | |
| | | E17387 | E17387 | |
| | | | | |
| | R₁ = H | X = -CH₃ | X = -CH₃ | 0,30 |
| | | | R₁ = H | |
| méthylsiliconate de sodium | R₂ = OH | | R₂ = OH | |
| --- | | | | |
| 6-déoxy-L-mannopyranose | R₃ = -CH₂OH | | R₃ = -CH₂OH | |
| | --- | δ = -37,26 ppm | δ = - 37,56 ppm | |
| | 30/70 | --- | --- | |
| | | E17483 | E17483 | |
| | | | | |
| | | X = -CH₃ | X = -CH₃ | 0,28 |
| méthylsiliconate de sodium | R₁ = H | | R₁ = H | |
| --- | R₂ = OH | | R₂ = OH | |
| acide théophylline acétique | R₃ = -CH₂OH | | R₃ = -CH₂OH | |
| | --- | | | |
| | 50/50 | δ = -37,00 ppm | δ = - 37,28 ppm | |
| | | --- | --- | |
| | | E17482 | E17482 | |
| | | | | |
| | | X = -CH₃ | X = -CH₃ | 0,51 |
| | R₁ = H | | R₂ = H | |
| méthylsiliconate de sodium | R₂ = H | | R₂ = H | |
| --- | R₃ = -CH₂OH | | R₃ = -CH₂OH | |
| hydroxyproline | --- | | | |
| | 50/50 | δ = -37,33 ppm | δ = - 37,84 ppm | |
| | | --- | --- | |
| | | E17388 | E17388 | |
| | | | | |
| | | X = -CH₂-CH₂-CH₂-CH₃ | X = -CH₂-CH₂-CH₂-CH₃ | 0,90 |
| | R₁ = H | | R₂ = H | |
| butyltrichloro-silane | R₂ = H | | R₂ = H | |
| --- | R₃ = -CH₂OH | | | |
| acide lactique | --- | | R₃ = -CH₂OH | |
| | 50/50 | δ = -38,24 ppm | δ = - 39,14 ppm | |
| | | --- | --- | |
| | | E38480 | E38480 | |
| | | | | |
| | | X = -CH₂OH | X = -CH₂OH | 1,15 |
| | R₁ = H | | R₁ = H | |
| hydroxyméthyl-triéthoxysilane | R₂ = H | | R₂ = H | |
| --- | R₃ = -CH₂OH | | | |
| caféine | --- | | R₃ = -CH₂OH | |
| | 50/50 | δ = -47,93 ppm | δ = - 49,08 ppm | |
| | | --- | --- | |
| | | E38478 | E38478 | |

Enfin, à titre d'illustrations finales, il est mentionné ci-après cinq exemples de formulation de composition selon l'invention contenant un silicium organique en solution hydroglycolique objet de l'invention précitée avec un descriptif utilisé pour la préparation de chaque silicium organique formulé.

### Formule A (crème)

Silicium organique en solution hydroglycolique (eau/1,3-propanediol ; 50/50) selon l'invention (à savoir le susnommé "complexe ternaire représenté par le schéma OS(I)---AS---OS(III)") impliquant les substances méthylsilanetriol, (3-hydroxypropoxy)-(méthyl)silanediol et l'adénosine au titre d'agent stabilisant/complexant, ledit silicium organique en solution

| | | |
|---|---|---|
| hydroglycolique étant préparé selon le test 1 décrit ci-avant | | 5 % |
| Polyisobutène hydrogéné | | 7 % |
| Myristate d'isobutyle | | 3 % |
| Palmitate de cétyle | | 7 % |
| Monostéarate d'éthylène glycol | | 5 % |
| Laurate sorbitan | | 2 % |
| Polysorbate 20 | | 2 % |
| Carbomer (copolymère d'acrylate/acrylamide & huile minérale) | | 0,3 % |
| Phénoxyéthanol | | 0,5 % |
| Eau | qsp 100% | |

### Formule B (qel)

Silicium organique en solution hydroglycolique (eau/1,3-propanediol ; 80/20) selon l'invention (à savoir le susnommé "complexe ternaire représenté par le schéma OS(I)---AS---OS(III)") impliquant les substances méthylsilanetriol, (3-hydroxypropoxy)-(méthyl)silanediol et N-acétyl-tyrosine au titre d'agent stabilisant/complexant, ledit silicium organique en solution hydroglycolique étant préparé selon le procédé de préparation décrit en test 1 ci-avant mais utilisant successivement 7,1 g de N-acétyl-tyrosine (32 mmoles) dans 690 g d'H₂O, 6,92 g d'une solution commerciale de méthylsiliconate de sodium (équivalent à 32 mmoles de méthylsilanetriol) diluée dans 40 q d'eau, 200 q de 1,3-propanediol puis ajustement masse par

| | |
|---|---|
| addition d'H₂O jusqu'à 1 kg | 6 % |
| Carbomer (copolymère d'acrylate/acrylamide & huile minérale) | 1,5 % |
| Benzoate de sodium | 0,2 % |
| Acide sorbique | 1 % |
| Soude | 0,13 % |
| Phénoxyéthanol | 0,9 % |
| Eau | qsp 100% |

### Formule C (lotion)

Silicium organique en solution hydroglycolique (eau/1,3-butanediol ; 50/50) selon l'invention (à savoir le susnommé "complexe ternaire représenté par le schéma OS(I)---AS---OS(III)") impliquant les substances méthylsilanetriol, (2-méthyl-3-hydroxypropoxy)-(méthyl)silanediol et le tréhalose au titre d'agent stabilisant/complexant, ledit silicium organique en solution hydroglycolique étant préparé selon le procédé de préparation décrit en test 1 ci-avant mais utilisant successivement 50 g de tréhalose (146 mmoles) dans 690 g d'H₂O, 6,92 g d'une solution commerciale de méthylsiliconate de sodium (équivalent à 32 mmoles de méthylsilanetriol) diluée dans 40 g d'eau, 500 g de 1,3-butanediol puis ajustement masse par

| | |
|---|---|
| addition d'H₂O jusqu'à 1 kg | 3 % |
| Chlorphénésine | 0,2 % |
| Phenonip (parabènes - parahydroxybenzoate de butyle, éthyle, | |
| isobutyle, méthyle, propyle et phénoxyéthanol) | 0,6 % |
| Gomme xanthique | 0,3 % |
| Triéthanolamine | 0,03% |
| Eau | qsp 100% |

### Formule D (émulsion)

Silicium organique en solution hydroglycolique (eau/1,2,3-propanetriol ; 30/70) selon l'invention (à savoir le susnommé "complexe ternaire représenté par le schéma OS(I)---AS-OS(III)") impliquant les substances méthylsilanetriol, (2,3-dihydroxypropoxy)-(méthyl)silanediol et L-arginine HCl au titre d'agent stabilisant/complexant, ledit silicium organique en solution hydroglycolique étant préparé selon le procédé de préparation décrit en test 1 ci-avant mais utilisant successivement 6,7 g de L-arginine HCl (32 mmoles), 6,92 g d'une solution commerciale de méthylsiliconate de sodium (équivalent à 32 mmoles de méthylsilanetriol) diluée dans 40 g d'eau, 700 g de 1,2,3-propanetriol puis ajustement masse

| | |
|---|---|
| par addition d'H₂O jusqu'à 1 kg | 8 % |
| Polydécène hydrogéné | 8 % |
| Triglycérides capriques/capriliques | 2 % |
| Oléate d'éthoxydiglycol | 8 % |
| Stéarate de glycéryle | 2 % |
| Diméthicone | 1 % |
| Stéarate de | |
| polyéthylène glycol-100 et stéarate de glycéryle | 5 % |
| Parabène de propyle | 0,3 % |
| Alcool stéarylique | 1 % |
| EDTA (éthylènediamine-sel disodique dihydraté de l'acide | |
| tétraacétique) | 0,2 % |
| Gomme xanthique | 0,4 % |
| Huile de germe de blé | 1 % |
| Huile de graines de Macadamia | 1 % |
| Polyéthylène glycol-8 & tocophérol | |
| & palmitate d'ascorbyle & acide ascorbique & acide citrique | 0,07% |
| Triéthanolamine | 0,35 % |
| Eau | qsp 100% |

### Formule E (complément alimentaire)

Silicium organique en solution hydroglycolique (eau/1,2,3-propanetriol ; 80/20) selon l'invention (à savoir le susnommé "complexe ternaire représenté par le schéma OS(I)---AS-OS(III)") impliquant les substances méthylsilanetriol, (2,3-dihydroxypropoxy)-(méthyl)silanediol et l'acide alginique au titre d'agent stabilisant/complexant, ledit silicium organique en solution hydroglycolique étant préparé selon le procédé de préparation décrit en test 1 ci-avant mais utilisant successivement 3,9 g d'acide alginique (18 mmoles), 6,92 g d'une solution commerciale de méthylsiliconate de sodium (équivalent à 32 mmoles de méthysilanetriol) diluée dans 40 g d'eau, 200 g de 1,2,3-propanetriol puis ajustement masse

| | |
|---|---|
| par addition d'H₂O jusqu'à 1 kg | 14 % |
| Sorbate de potassium | 0,05 % |
| Benzoate de sodium | 0,1 % |
| Arômes | 0,03 % |
| Eau | qsp 100% |

## Revendications

1. Composition hydroglycolique comprenant :
a) un premier organo-silanol de formule générale (I) suivante :
X-Si(OH)₃ (I)
dans laquelle le radical X est un groupement alkyle en C₁-C₄, linéaire ou ramifié, de préférence en C₁-C₂, éventuellement substitué par au moins un groupement hydroxyle, de préférence X étant un groupement méthyle ;
b) un deuxième organo-silanol de formule générale (III) suivante :
X-Si(OH)₂-O-CH(R₁)-CH(R₂)(R₃) (III)
dans laquelle :
- le radical X est un groupement alkyle en C₁-C₄, linéaire ou ramifié, de préférence en C₁-C₂, éventuellement substitué par au moins un groupement hydroxyle, de préférence X étant un groupement méthyle ;
- R₁ est -H ou -CH₃;
- R₂ est -H, -OH, ou -CH₃;
- R₃ est -CH₃, -CH₂-CH₃, -CH₂OH, -CH(CH₃)OH, -CH₂-CH₂-CH₃, -CH₂-CH₂-CH₂-CH₃, -CH₂-CH₂-CH₂-CH₂-OH, ou -C(CH₃)(CH₃)-OH ; et
c) un agent stabilisant d'organo-silanol(s), adapté pour permettre la formation d'un complexe moléculaire avec au moins un organo-silanol via l'établissement d'au moins une liaison chimique faible, préférablement d'au moins une liaison hydrogène, avec ledit au moins un organo-silanol.

2. Composition selon la revendication 1, dans laquelle le rapport molaire entre ledit premier organo-silanol et ledit deuxième organo-silanol est compris entre 100/1 et 100/20, de préférence entre 100/10 et 100/15.

3. Composition selon la revendication 1 ou 2, ladite composition étant une solution hydroglycolique.

4. Composition selon l'une des revendications précédentes, dans laquelle ledit au moins un solvant glycolique répond à la formule générale (II) suivante :
HO-CH(R₁)-CH(R₂)(R₃) (II)
dans laquelle :
- R₁ est -H ou -CH₃ ;
- R₂ est -H, -OH, ou -CH₃;
- R₃ est -CH₃, -CH₂-CH₃, -CH₂OH, -CH(CH₃)OH, -CH₂-CH₂-CH₃, -CH₂-CH₂-CH₂-CH₃, -CH₂-CH₂-CH₂-CH₂-OH, ou -C(CH₃)(CH₃)-OH.

5. Composition selon l'une des revendications précédentes, dans laquelle :
i) le rapport massique eau/solvant glycolique est compris entre 98/2 et 20/80, de préférence entre 80/20 et 20/80, avantageusement entre 80/20 et 50/50, et/ou
ii) le pH de ladite composition est compris entre environ 4 et environ 6 ;
de manière préférée, ladite composition présentant les caractéristiques i) et ii).

6. Composition selon l'une des revendications précédentes, dans laquelle ledit agent stabilisant d'organo-silanol(s) est une substance biologiquement active, préférablement choisie parmi l'adénosine, l'acide glyccyrrhétinique et/ou ses sels, la glycyrrhizine et/ou ses sels, l'acide lactobionique et/ou ses sels, l'acide alginique et/ou ses sels, l'acide hyaluronique et/ou ses sels, le lactose, le tréhalose, le 6-déoxy-L-mannopyranose et/ou ses sels, l'acide théophylline acétique, l'acide ascorbique, l'acide lactique, l'acide salicylique et/ou ses sels, l'acide pyrrolidone carboxylique et/ou ses sels ; l'arginine, la sérine, la lysine et/ou leurs sels, la méthionine et/ou ses sels, l'acétyl-méthionine, la thréonine, l'hydroxyproline, la N-acétyl-tyrosine, l'acide aspartique, l'acide glutamique et/ou leurs sels, l'alcool oléique, le panthénol, la caféine, la pectine, l'acéfylline, le sulfate de chondroïtine et/ou leurs sels, la perle hydrolysée, un hydrolysat de protéines d'origine animale ou végétale, par exemple un hydrolysat de collagène d'origine marine tel qu'un hydrolysat de peau(x) de poisson(s), et leurs mélanges, avantageusement choisie parmi l'acide alginique et/ou ses sels, le 6-déoxy-L-mannopyranose et/ou ses sels, l'acide hyaluronique et/ou ses sels, l'acide ascorbique, l'hydroxyproline, l'acide théophylline acétique, l'acide salicylique et/ou ses sels, l'adénosine, la caféine, et leurs mélanges, de manière particulièrement préférée choisie parmi l'acide alginique et/ou ses sels ou l'acide hyaluronique et/ou ses sels.

7. Composition selon l'une des revendications précédentes, ledit deuxième organo-silanol de formule générale (III) étant défini comme suit :
X-Si(OH)₂-O-CH(R₁)-CH(R₂)(R₃) (III)
dans laquelle :
- X est un groupement méthyle ;
- R₁ est un atome d'hydrogène ;
- R₂ est un atome d'hydrogène ; et
- R₃ est -CH₂-OH.

8. Composition pharmaceutique, médicament à usage humain ou vétérinaire, dispositif médical tel qu'une solution injectable, complément alimentaire à usage humain ou animal, composition cosmétique, composition dermo-cosmétique, comprenant la composition selon l'une des revendications précédentes.

9. Composition selon l'une des revendications 1 à 7 pour son utilisation en tant que médicament à usage humain ou vétérinaire.

10. Composition selon l'une des revendications 1 à 7 pour son utilisation :
- dans la prévention et/ou la limitation des dégradations cutanées liées au vieillissement de la peau, par exemple liées à un stress oxydatif générant des radicaux libres ou des espèces réactives de l'oxygène,
- pour relancer l'activité cellulaire épidermique, dermique et/ou hypodermique, et/ou
- comme agent stimulant sur l'expression de collagènes fibrillaires et protéoglycanes constitutifs de la jonction dermo-épidermique.

11. Procédé de préparation d'un organo-silanol de formule générale (III) tel que défini dans l'une des revendications 1 à 7, comprenant les étapes suivantes :
a) obtenir un organo-silanol de formule générale (I) suivante :
X-Si(OH)₃ (I)
dans laquelle le radical X est un groupement alkyle en C₁-C₄, linéaire ou ramifié, de préférence en C₁-C₂, éventuellement substitué par au moins un groupement hydroxyle, de préférence X étant un groupement méthyle ;
b) mettre en contact ledit organo-silanol de formule générale (I) avec au moins un solvant glycolique répondant à la formule générale (II) suivante :
HO-CH(R₁)-CH(R₂)(R₃) (II)
dans laquelle :
- R₁ est -H ou -CH₃ ;
- R₂ est -H, -OH, ou -CH₃;
- R₃ est -CH₃, -CH₂-CH₃, -CH₂OH, -CH(CH₃)OH, -CH₂-CH₂-CH₃, -CH₂-CH₂-CH₂-CH₃, -CH₂-CH₂-CH₂-CH₂-OH, ou -C(CH₃)(CH₃)-OH ;
durant un laps de temps suffisant pour obtenir, via une réaction de monoalcoxylation, la formation dudit organo-silanol de formule générale (III).

12. Utilisation d'un organo-silanol de formule générale (III) tel que défini dans l'une des revendications 1 à 7, pour accroître la stabilité d'une composition comprenant :
- un organo-silanol de formule générale (I) suivante :
X-Si(OH)₃ (I)
dans laquelle le radical X est un groupement alkyle en C₁-C₄, linéaire ou ramifié, de préférence en C₁-C₂, éventuellement substitué par au moins un groupement hydroxyle, de préférence X étant un groupement méthyle ; et
- un agent stabilisant d'organo-silanol(s) tel que défini dans la revendication 1 ou 6.

13. Utilisation selon la revendication 12, ledit organo-silanol de formule générale (III) étant défini comme suit :
X-Si(OH)₂-O-CH(R₁)-CH(R₂)(R₃) (III)
dans laquelle :
- X est un groupement méthyle ;
- R₁ est un atome d'hydrogène ;
- R₂ est un atome d'hydrogène ; et
- R₃ est -CH₂-OH.

14. Utilisation d'une composition selon l'une quelconque des revendications 1 à 7, comme ingrédient actif cosmétique ou dermocosmétique, ou pour la fabrication de préparations ou de compléments alimentaires, diététiques, cosmétiques ou pharmaceutiques à usage humain ou animal.

15. Utilisation selon la revendication 14, ladite composition étant utilisée comme ingrédient cosmétique adapté pour réduire la taille et/ou la visibilité des pores visibles de la peau liés à l'âge.

## Patentansprüche

1. Eine hydroglykolische Zusammensetzung, die Folgendes beinhaltet:
a) ein erstes Organosilanol der folgenden allgemeinen Formel (I):
X-Si(OH)₃ (I)
wobei der Rest X eine lineare oder verzweigte C₁-C₄-Alkylgruppe, vorzugsweise C₁-C₂, ist, die optional mit mindestens einer Hydroxylgruppe substituiert ist, wobei X vorzugsweise eine Methylgruppe ist;
b) ein zweites Organosilanol der folgenden allgemeinen Formel (III):
X-Si(OH)₂-O-CH(R₁)-CH(R₂)(R₃) (III)
wobei:
- der Rest X eine lineare oder verzweigte C₁-C₄-Alkylgruppe, vorzugsweise C₁-C₂, ist, die optional mit mindestens einer Hydroxylgruppe substituiert ist, wobei X vorzugsweise eine Methylgruppe ist;
- R₁ -H oder -CH₃ ist;
- R₂ -H, -OH oder -CH₃ ist;
- R₃ -CH₃, -CH₂-CH₃, -CH₂OH, -CH(CH₃)OH, -CH₂-CH₂-CH₃, -CH₂-CH₂-CH₂-CH₃, -CH₂-CH₂-CH₂-CH₂-OH oder -C(CH₃)(CH₃)-OH ist; und
c) ein Organosilanol(e)-Stabilisierungsmittel, das angepasst ist, um die Bildung eines molekularen Komplexes mit mindestens einem Organosilanol über die Erstellung von mindestens einer schwachen chemischen Bindung, vorzugsweise mindestens einer Wasserstoffbindung, mit dem mindestens einen Organosilanol zu ermöglichen.

2. Zusammensetzung gemäß Anspruch 1, wobei das Molverhältnis zwischen dem ersten Organosilanol und dem zweiten Organosilanol zwischen 100 : 1 und 100 : 20, vorzugsweise zwischen 100 : 10 und 100 : 15, liegt.

3. Zusammensetzung gemäß Anspruch 1 oder 2, wobei die Zusammensetzung eine Hydroglykollösung ist.

4. Zusammensetzung gemäß einem der vorhergehenden Ansprüche, wobei das mindestens eine Glykollösungsmittel der folgenden allgemeinen Formel (II) entspricht:
HO-CH(R₁)-CH(R₂)(R₃) (II)
wobei:
- R₁ -H oder -CH₃ ist;
- R₂ -H, -OH oder -CH₃ ist;
- R₃ -CH₃, -CH₂-CH₃, -CH₂OH, -CH(CH₃)OH, -CH₂-CH₂-CH₃, -CH₂-CH₂-CH₂-CH₃, -CH₂-CH₂-CH₂-CH₂-OH oder -C(CH₃)(CH₃)-OH ist.

5. Zusammensetzung gemäß einem der vorhergehenden Ansprüche, wobei:
i) das Massenverhältnis von Wasser zu Glykollösungsmittel zwischen 98 : 2 und 20 : 80, vorzugsweise zwischen 80 : 20 und 20 : 80, vorteilhafterweise zwischen 80 : 20 und 50 : 50, liegt, und/oder
ii) der pH-Wert der Zusammensetzung zwischen etwa 4 und etwa 6 liegt;
wobei die Zusammensetzung vorzugsweise die Charakteristika i) und ii) darstellt.

6. Zusammensetzung gemäß einem der vorhergehenden Ansprüche, wobei das Organosilanol(e)-Stabilisierungsmittel eine biologisch aktive Substanz ist, die vorzugsweise aus Folgendem ausgewählt ist: Adenosin, Glycyrrhetinsäure und/oder deren Salzen, Glycyrrhizin und/oder dessen Salzen, Lactobionsäure und/oder deren Salzen, Alginsäure und/oder deren Salzen, Hyaluronsäure und/oder deren Salzen, Lactose, Trehalose, 6-Deoxy-L-Mannopyranose und/oder deren Salzen, Theophyllin-Essigsäure, Ascorbinsäure, Milchsäure, Salicylsäure und/oder deren Salzen, Pyrrolidoncarbonsäure und/oder deren Salzen; Arginin, Serin, Lysin und/oder deren Salzen, Methionin und/oder dessen Salzen, Acetyl-Methionin, Threonin, Hydroxyprolin, N-Acetyl-Tyrosin, Asparaginsäure, Glutaminsäure und/oder deren Salzen, Ölalkohol, Panthenol, Koffein, Pektin, Acefyllin, Chondroitinsulfat und/oder dessen Salzen, hydrolysierter Perle, einem Proteinhydrolysat tierischen oder pflanzlichen Ursprungs, zum Beispiel einem Kollagenhydrolysat marinen Ursprungs wie einem Hydrolysat aus Fischhaut/-häuten, und deren Mischungen, vorteilhafterweise ausgewählt aus Alginsäure und/oder deren Salzen, 6-Deoxy-L-Mannopyranose und/oder deren Salzen, Hyaluronsäure und/oder deren Salzen, Ascorbinsäure, Hydroxyprolin, Theophyllin-Essigsäure, Salicylsäure und/oder deren Salzen, Adenosin, Koffein, und deren Mischungen, besonders bevorzugt ausgewählt aus Alginsäure und/oder deren Salzen oder Hyaluronsäure und/oder deren Salzen.

7. Zusammensetzung gemäß einem der vorhergehenden Ansprüche, wobei das zweite Organosilanol der allgemeinen Formel (III) wie folgt definiert ist:
X-Si(OH)₂-O-CH(R₁)-CH(R₂)(R₃) (III)
wobei:
- X eine Methylgruppe ist;
- R₁ ein Wasserstoffatom ist;
- R₂ ein Wasserstoffatom ist; und
- R₃ -CH₂-OH ist.

8. Eine pharmazeutische Zusammensetzung, ein Arzneimittel zur Verwendung an Mensch oder Tier, eine medizinische Vorrichtung wie eine Injektionslösung, ein Nahrungsergänzungsmittel für die Verwendung bei Mensch oder Tier, eine kosmetische Zusammensetzung, eine dermo-kosmetische Zusammensetzung, die die Zusammensetzung gemäß einem der vorhergehenden Ansprüche beinhaltet.

9. Zusammensetzung gemäß einem der Ansprüche 1 bis 7 zur Verwendung als Arzneimittel für die Verwendung bei Mensch oder Tier.

10. Zusammensetzung gemäß einem der Ansprüche 1 bis 7 zur Verwendung für Folgendes:
- bei der Prävention und/oder Begrenzung von Hautschädigungen im Zusammenhang mit der Hautalterung, zum Beispiel im Zusammenhang mit oxidativem Stress, der freie Radikale oder reaktive Sauerstoffspezies erzeugt,
- zur Wiederbelebung der epidermalen, dermalen und/oder hypodermalen Zellaktivität und/oder
- als Stimulationsmittel für die Expression von fibrillären Kollagenen und Proteoglykanen, die für die dermo-epidermale Verbindung konstitutiv sind.

11. Ein Verfahren zur Herstellung eines Organosilanols der allgemeinen Formel (III), wie in einem der Ansprüche 1 bis 7 definiert, das die folgenden Schritte beinhaltet:
a) Erhalten eines Organosilanols der folgenden allgemeinen Formel (I):
X-Si(OH)₃ (I)
wobei der Rest X eine lineare oder verzweigte C₁-C₄-Alkylgruppe, vorzugsweise C₁-C₂, ist, die optional mit mindestens einer Hydroxylgruppe substituiert ist, wobei X vorzugsweise eine Methylgruppe ist;
b) In-Kontakt-Bringen des Organosilanols der allgemeinen Formel (I) mit mindestens einem Glykollösungsmittel, das der folgenden allgemeinen Formel (II) entspricht:
HO-CH(R₁)-CH(R₂)(R₃) (II)
wobei:
- R₁ -H oder -CH₃ ist;
- R₂ -H, -OH oder -CH₃ ist;
- R₃ -CH₃, -CH₂-CH₃, -CH₂OH, -CH(CH₃)OH, -CH₂-CH₂-CH₃, -CH₂-CH₂-CH₂-CH₃, -CH₂-CH₂-CH₂-CH₂-OH oder -C(CH₃)(CH₃)-OH ist;
während eines Zeitraums, der ausreicht, um über eine Monoalkoxylierungsreaktion die Bildung des Organosilanols der allgemeinen Formel (III) zu erhalten.

12. Eine Verwendung eines Organosilanols der allgemeinen Formel (III), wie in einem der Ansprüche 1 bis 7 definiert, zur Erhöhung der Stabilität einer Zusammensetzung, die Folgendes beinhaltet:
- ein Organosilanol der folgenden allgemeinen Formel (I):
X-Si(OH)₃ (I)
wobei der Rest X eine lineare oder verzweigte C₁-C₄-Alkylgruppe, vorzugsweise C₁-C₂, ist, die optional mit mindestens einer Hydroxylgruppe substituiert ist, wobei X vorzugsweise eine Methylgruppe ist; und
- ein Organosilanol(e)-Stabilisierungsmittel, wie in Anspruch 1 oder 6 definiert.

13. Verwendung gemäß Anspruch 12, wobei das Organosilanol der allgemeinen Formel (III) wie folgt definiert ist:
X-Si(OH)₂-O-CH(R₁)-CH(R₂)(R₃) (III)
wobei:
- X eine Methylgruppe ist;
- R₁ ein Wasserstoffatom ist;
- R₂ ein Wasserstoffatom ist; und
- R₃ -CH₂-OH ist.

14. Verwendung einer Zusammensetzung gemäß einem der Ansprüche 1 bis 7 als kosmetischen oder dermo-kosmetischen Wirkstoff oder zur Fertigung von Nahrungsmittel-, diätetischen, kosmetischen oder pharmazeutischen Präparaten oder Ergänzungsmitteln für die Verwendung bei Mensch oder Tier.

15. Verwendung gemäß Anspruch 14, wobei die Zusammensetzung als kosmetischer Inhaltsstoff verwendet wird, der zur Verringerung der Größe und/oder Sichtbarkeit von altersbedingten sichtbaren Poren der Haut angepasst ist.

## Claims

1. A hydroglycolic composition comprising:
a) a first organo-silanol of the following general formula (I):
X-Si(OH)₃ (I)
wherein the radical X is a linear or branched C₁-C₄ alkyl group, preferably C₁-C₂, possibly substituted with at least one hydroxyl group, with preferably X being a methyl group;
b) a second organo-silanol of the following general formula (III):
X-Si(OH)₂-O-CH(R₁)-CH(R₂)(R₃) (III)
wherein:
- the radical X is a linear or branched C₁-C₄ alkyl group, preferably C₁-C₂, possibly substituted with at least one hydroxyl group, with preferably X being a methyl group;
- R₁ is -H or -CH₃;
- R₂ is -H, -OH, or-CH₃;
- R₃ is -CH₃, -CH₂-CH₃, -CH₂OH, -CH(CH₃)OH, -CH₂-CH₂-CH₃, -CH₂-CH₂-CH₂-CH₃, -CH₂-CH₂-CH₂-CH₂-OH, or -C(CH₃)(CH₃)-OH; and
c) an organo-silanol(s) stabilising agent, suitable for allowing the formation of a molecular complex with at least one organo-silanol via the establishment of at least one weak chemical bond, preferably at least one hydrogen bond, with said at least one organo-silanol.

2. The composition according to claim 1, wherein the molar ratio between said first organo-silanol and said second organo-silanol is between 100/1 and 100/20, preferably between 100/10 and 100/15.

3. The composition according to claim 1 or 2, said composition being a hydroglycolic solution.

4. The composition according to any one of the preceding claims, wherein said at least one glycolic solvent has the following general formula (II):
HO-CH(R₁)-CH(R₂)(R₃) (II)
wherein:
- R₁ is -H or -CH₃;
- R₂ is -H, -OH, or-CH₃;
- R₃ is -CH₃, -CH₂-CH₃, -CH₂OH, -CH(CH₃)OH, -CH₂-CH₂-CH₃, -CH₂-CH₂-CH₂-CH₃, -CH₂-CH₂-CH₂-CH₂-OH, or -C(CH₃)(CH₃)-OH.

5. The composition according to any one of the preceding claims, wherein:
i) the water/glycol solvent mass ratio is between 98/2 and 20/80, preferably between 80/20 and 20/80, advantageously between 80/20 and 50/50, and/or
ii) the pH of said composition is between about 4 and about 6;
with preferably said composition presenting characteristics i) and ii).

6. The composition according to any one of the preceding claims, wherein said organo-silanol(s) stabilising agent is a biologically active substance, preferably selected from adenosine, glycyrrhizic acid and/or its salts, glycyrrhizin and/or its salts, lactobionic acid and/or its salts, alginic acid and/or its salts, hyaluronic acid and/or its salts, lactose, trehalose, 6-deoxy-L-mannopyranose and/or its salts, theophylline acetic acid, ascorbic acid, lactic acid, salicylic acid and/or its salts, pyrrolidone carboxylic acid and/or its salts, arginine, serine, lysine and/or their salts, methionine and/or its salts, acetyl methionine, threonine, hydroxyproline, N-acetyl tyrosine, aspartic acid, glutamic acid and/or their salts, oleic alcohol, panthenol, caffeine, pectin, acefylline, chondroitin sulfate and/or their salts, hydrolysed pearl, a protein hydrolysate of animal or plant origin, for example a collagen hydrolysate of marine origin such as a collagen hydrolysate from fish skin(s), and mixtures thereof, advantageously chosen from alginic acid and/or its salts, 6-deoxy-L-mannopyranose and/or its salts, hyaluronic acid and/or its salts, ascorbic acid, hydroxyproline, theophylline acetic acid, salicylic acid and/or its salts, adenosine, caffeine, and mixtures thereof, particularly preferably chosen from alginic acid and/or its salts, or hyaluronic acid and/or its salts.

7. The composition according to any one of the preceding claims, said second organo-silanol of general formula (III) being defined as follows:
X-Si(OH)₂-O-CH(R₁)-CH(R₂)(R₃) (III)
wherein:
- X is a methyl group;
- R₁ is a hydrogen atom;
- R₂ is a hydrogen atom; and
- R₃ is -CH₂₋OH.

8. A pharmaceutical composition, drug for human or veterinary use, medical device such as an injectable solution, food supplement for human or animal use, cosmetic composition, dermocosmetic composition, comprising the composition according to any one of the preceding claims.

9. The composition according to any one of claims 1 to 7 for its use as a drug for human or veterinary use.

10. The composition according to any one of claims 1 to 7 for its use:
- in the prevention and/or limitation of skin damage related to skin ageing, for example related to oxidative stress generating free radicals or reactive oxygen species,
- for boosting the epidermal, dermal and/or hypodermal cellular activity, and/or
- as a stimulating agent on the expression of fibrillar collagens and proteoglycans constituting the dermo-epidermaljunction.

11. Process for preparing an organosilanol of general formula (III) as defined in any one of claims 1 to 7, comprising the following steps:
a) obtaining an organo-silanol of the following general formula (I):
X-Si(OH)₃ (I)
wherein the radical X is a linear or branched C₁-C₄ alkyl group, preferably C₁-C₂, possibly substituted with at least one hydroxyl group, with preferably X being a methyl group;
b) contacting said organo-silanol of general formula (I) with at least one glycolic solvent corresponding to the following general formula (II):
HO-CH(R₁)-CH(R₂)(R₃) (II)
wherein:
- R₁ is -H or -CH₃;
- R₂ is -H, -OH, or-CH₃;
- R₃ is -CH₃, -CH₂-CH₃, -CH₂OH, -CH(CH₃)OH, -CH₂-CH₂-CH₃, -CH₂-CH₂-CH₂-CH₃, -CH₂-CH₂-CH₂-CH₂-OH, or -C(CH₃)(CH₃)-OH;
for a sufficient period of time to obtain, via a mono-alkoxylation reaction, the formation of the above-mentioned organo-silanol of general formula (III).

12. Use of an organo-silanol of general formula (III) as defined in any one of claims 1 to 7, for increasing the stability of a composition comprising:
- an organo-silanol of the following general formula (I):
X-Si(OH)₃ (I)
wherein the radical X is a linear or branched C₁-C₄ alkyl group, preferably C₁-C₂, possibly substituted with at least one hydroxyl group, with preferably X being a methyl group; and
- an organo-silanol(s) stabilising agent as defined in claim 1 or 6.

13. The use according to claim 12, with said organo-silanol of general formula (III) being defined as follows:
X-Si(OH)₂-O-CH(R₁)-CH(R₂)(R₃) (III)
wherein:
- X is a methyl group;
- R₁ is a hydrogen atom;
- R₂ is a hydrogen atom; and
- R₃ is -CH₂-OH.

14. The use of a composition according to any one of claims 1 to 7, as a cosmetic or dermocosmetic active ingredient, or for the manufacture of food, dietetic, cosmetic or pharmaceutical preparations or supplements for human or animal use.

15. The use according to claim 14, said composition being used as a cosmetic ingredient suitable for reducing the size and/or visibility of noticeable skin pores associated with age.
